# EUROPEAN PATENT APPLICATION

(11) **EP 4 571 771 A1**
(43) Date of publication of application: **18.06.2025**
(21) Application number: 23216963.1
(22) Date of filing: 15.12.2023
(51) Int. Cl.: G16H 40/40

(54) **CLINICAL WORKFLOW TRANSFORMATION**

(71) Applicant: Koninklijke Philips N.V., 5656 AG Eindhoven (NL)
(72) Inventor: BOUWMAN, Wilbert, Eindhoven (NL); BULUT, Murtaza, Eindhoven (NL); RASMIJN, Anita, 5656AG Eindhoven (NL)
(74) Representative: Philips Intellectual Property & Standards

(57) **Abstract**

The invention provides a system and method for generating proposed modifications to clinical workflows for transferring monitoring steps which are, by default, performed on-site in a clinical setting such as a hospital to an off-site setting such as the home of the patient, a remote clinic, or a nursing home. To this end, it is proposed to review workflow steps of a set of workflows and identify steps which involve acquisition of measurement data for a patient and to identify off-site, portable measurement devices from an inventory list whose data acquisition capabilities are sufficient to acquire the needed measurement data for that step. A corresponding workflow step transformation suggestion is generated.

## Description

### FIELD OF THE INVENTION

The present invention relates generally to the field of patient monitoring and treatment.

### BACKGROUND OF THE INVENTION

One function that may be performed by clinical decision support (CDS) systems is to support care providers in following pre-defined clinical workflows in the monitoring and treatment of patients. A workflow may be defined by a workflow specification which defines a plurality of steps linked by a process flow structure, e.g. defining an order of steps and possibly defining a conditional logic structure linking steps. A clinical decision support system may track progress or status within a workflow for a patient and support a clinician in automatically caching and trending relevant biological parameters at appropriate times and in prompting or triggering patient interaction events, to be performed either by a clinician or by a patient interaction device such as a sensing/measurement device or a treatment device (e.g. an IV).

Within this context, an important factor is the management and optimization of the workflows whose implementation is being supported by the CDS system.

With regards to the construction and design of clinical workflows, the underlying reasoning and logic can originate from a number of different sources, as will now be briefly discussed.

Clinical practice guidelines (CPGs) are used for clinical decision making and as a standard framework for measuring quality of care. They can be defined as "systematically developed statements to assist practitioner and patient decisions about appropriate health care for specific clinical circumstances" (Field et.al., Institute of Medicine, National Academies Press; 1990).

Hospital protocols are procedural descriptions of how CPGs are applied. They are the guidelines used by the institution to provide care and describe what is being done. They can contain workflows.

Clinical workflows are a series of interconnected events and tasks, performed by different professionals (agents), at multiple levels that can work simultaneously or sequentially to achieve a particular goal, as described in a protocol.

Having a pattern that can be relied upon, as part of health service delivery, is valuable. This minimizes the occurrence of mistakes and complications. A workflow may be understood as an actionable implementation of processes with the aim of improving productivity and assuring outcomes.

Monitoring of vital signs on patients is often one of the steps in a workflow. Monitoring is for example applicable for post-surgery, after a seizure or in case of other heart related conditions.

In a hospital environment, the measurement devices and monitoring are set up for the workflow to optimally monitor the patient.

Pre-defined alarms may be configured to detect when monitored clinical parameters exceed normal limits. For example there may be set an upper and a lower limit alarm.

Alarm detection functionality may be built into measurement devices themselves, and/or software-based alarms may be incorporated into a patient monitoring and/or decision support system. For example, a measurement device may be communicatively connected to a monitoring or telemetry system by which data is streamed or batch-transferred to a monitoring or decision support system which compiles and analyzes data in real time.

In some cases, such systems may include so-called "smart alarms" which include an additional layer of analysis for determining whether a triggered alarm is of high priority for attention of the clinician. A smart alarm can for example be a peak alarm, duration alarm, or combination of parameters alarm. Smart alarms can be based on best-practice clinical guidelines, published literature and/or clinical trials.

Most measurement devices designed for use inside a hospital environment are not easily transferred to use outside the hospital e.g., in an at home environment, due to specific handling and setup requirements.

Mobile or at home devices exist which can measure vital signs and other clinical parameters in a home environment. However, clinical workflows are not typically set up to utilize such devices, since workflows assume hospital implementation. Furthermore, such home-based devices may not be configured or setup with the particular settings needed for the monitoring requirements of a specific workflow.

It would be of value to enable more efficient use of home-based measurement devices as part of execution of clinical workflows.

### SUMMARY OF THE INVENTION

It is the recognition of the inventors that non-hospital measurement systems have the potential to improve patient care when fully integrated into the clinical practice (e.g., when a clinician responsible for a patient's care has the option to execute specific clinical protocol steps at home). In current practice, healthcare professionals view non -hospital measurement systems as disruptive to clinical practice since they are not fully integrated into workflow management systems, and their data output is not always trusted since data collection parameters and data quality is not always trustworthy.

It is the recognition of the inventors that effective use of home-based monitoring devices may be facilitated by providing means for ensuring that a selected home measurement device is in alignment with data acquisition needs of a particular workflow step to which it is to be applied, for example in terms of type of data, and preferably data quality and data acquisition characteristics. It would also be advantageous for configuration settings of the device be aligned with hospital practice (e.g., home alarm triggers linked to hospital alarm triggers).

The invention is defined by the claims.

According to examples in accordance with an aspect of the invention, there is provided a system comprising:
a workflow datastore storing a plurality of clinical workflow specifications. Each workflow specification may define a plurality of workflow steps. The workflow steps may be linked by a process flow structure. Each workflow step may be associated in the specification with a set of workflow step properties or components, wherein, for at least a subset of workflow steps, the workflow step properties include an indication of measurement data input requirements for the step and an indication of a patient interaction device (e.g. a patient measurement device) to be used for acquiring the measurement data.

The system may preferably further comprise a measurement device inventory recording a list of available patient measurement devices for use in acquiring measurement data. Each measurement device may be labelled with associated data acquisition capabilities and labelled as being a device which is either for on-site use or off-site use. On-site may mean a clinical environment, e.g. a hospital environment. Off-site may mean a non-clinical environment, e.g. a non-hospital environment, e.g. a home environment of the patient. On-site may mean on the same site as a clinical decision support system.

The system may further comprise a workflow transformation subsystem for generating workflow step transformation suggestions. Each step transformation suggestion may indicate at least one possible step of a workflow specification which may be transformed from an on-site step to an off-site step. The generating the workflow step transformation may include the following steps: accessing the workflow datastore; accessing the measurement device inventory; for each of one or more workflow steps of one or more workflows which are currently associated with use of an onsite device: reading the input data requirements associated with the step; comparing the input data requirements against data acquisition capabilities of one or more measurement devices listed in the inventory; identifying an offsite measurement device in the inventory for which the data acquisition capabilities match the input data requirements; and generating a workflow step transformation suggestion indicative of the identified offsite measurement device from the inventory, and the workflow step and workflow for which the device has been identified. The method may further comprise controlling a graphical user interface to provide an indication of generated workflow step transformation suggestion(s).

Thus, embodiments of the invention provide a system for adapting clinical workflow specifications in real time to accommodate transitioning steps of the workflow to a different site, therefore enabling balancing of demand at an on-site clinical setting.

In some embodiments, the workflow transformation subsystem is further configured to, for at least one workflow step transformation suggestion: replace the measurement device property of the workflow step with the offsite measurement device identified from the inventory; and output the modified workflow step.

In some embodiments, the system may further comprise alarm monitoring functionality for monitoring received measurement data from one or more of the measurement devices and comparing it against one or more alarm conditions. For example, the system may include an alarm monitoring module adapted to monitor received measurement data and compare it against one or more alarm conditions or criteria. The alarm conditions or criteria may for example include one or more alarm thresholds.

In some embodiments, one or more of the workflows in the workflow store include at least one step of monitoring a first physiological parameter of a patient and include an indication of at least one alarm condition in relation to the physiological parameter. The workflow step transformation may further include modifying the at least one alarm condition.

In some embodiments, the modifying of the one or more alarm conditions may be based on the step of comparing the input data requirements of the relevant workflow step with the data acquisition capabilities of the one or more measurement devices listed in the inventory. In other words, the modification of the at least one alarm condition may be determined based on the comparison between the input data requirements of the workflow step and the data acquisition capabilities of one or more measurement devices listed in the inventory. It may alternatively be based on use of a lookup table or database, wherein a proposed change in measurement device from a first device to a second device can be mapped to a corresponding one or more changes in the alarm conditions.

In some embodiments, the system may include a data collection module, e.g. a telemetry module, operable to communicate with offsite measurement devices via a communication channel to collect measurement data acquired by one or more off-site measurement devices. The data collection module may be configured to transfer measurement data acquired by off-site measurement devices from the data collection module to a decision support subsystem and/or a workflow hosting platform of the system.

The decision support subsystem and/or the workflow hosting platform may be configured to store, e.g. cache, the received measurement data for use in deriving trends.

In some embodiments, the system may include a plurality of off-site measurement devices. Each offsite measurement device may include functionality for exporting acquired measurement data via a direct or indirect communication channel to the data collection module. For example, each offsite measurement device may include a communication module for this purpose.

In some embodiments, the system may further comprise a data transformation module (e.g. a data transfer function) for: receiving measurement data acquired by an offsite measurement device and applying a data transformation function (e.g. a data transfer function) for modifying the received data to improve a match between the acquired data and data input requirements of at least one workflow step of a workflow.

In some embodiments, the input data requirements for each workflow step may include an indication of: a type of measurement data, and one or more data quality characteristics/specifications. The data acquisition capabilities recorded in the inventory for each measurement device may include an indication of measurement data type and data quality characteristics/specifications. The comparing step may comprise comparing the required data quality characteristics against the data quality characteristics of each measurement device.

In some embodiments, responsive to detecting a match in the measurement data type and a mismatch in the data quality characteristics, the workflow transformation subsystem may be configured to determine a transfer function for transforming measurement data acquired by the off-site measurement device into transformed measurement data having data quality characteristics better matching the required characteristics.

In some embodiments, the system further comprises measurement acquisition check functionality, configured to: receive measurement data from an offsite measurement device indicated by a workflow transformation suggestion, during a trial measurement session; evaluate one or more pre-defined data quality characteristics of the data; and generate feedback based on the evaluation. In other words, a trial is run to determine the degree of match between data quality of the offsite measurement device and the data quality requirements.

In some embodiments, the system may further comprise a user interface. The user interface may comprise a graphical user interface.

In some embodiments, the graphical user interface may be controlled to simultaneously provide, for a given workflow transformation suggestion, a visual representation of: the original workflow to which the workflow transformation suggestion relates and the suggested workflow transformation. In this way, a user is able to understand the proposed transformation in context of the original workflow.

In some embodiments, the method may further comprise determining a set of configuration settings for the offsite measurement device assigned to the workflow step. When outsourcing a workflow step to be performed by an offsite device, it may be valuable to configure settings of the device to optimize a match between data acquired and data acquisition requirements. This may for example comprise configuring data acquisition frequency, data resolution, device sensitivity as well as settings for communicating acquired measurement data with the decision support system at the hospital site.

In some embodiments, the system further comprises a workflow hosting platform for hosting one or more workflows, the workflow hosting platform comprising functionality for tracking progress through steps of each workflow being hosted. For example, the workflow hosting platform may receive measurement data from one or more measurement devices and use this in tracking progress through steps of one or more workflows.

In some embodiments, the system further comprises functionality for triggering one or more patient interaction events specified by steps of a workflow for acquiring measurement data pertaining to a patient. A patient interaction event may for example comprise an event for obtaining information about a current patient status or administering or adjusting a therapeutic action associated with a patient. Obtaining information about a current patient status may comprise use of a measurement device, e.g. one of the measurement devices recorded in the inventory, to acquire patient measurement data. A patient interaction event can in general be performed either by a clinician, optionally using a tool such as a measurement device, or directly by a patient interaction device through electronic triggering by the system, such as by a sensing/measurement device or a treatment device (e.g. an IV).

In some embodiments, the triggering of a patient interaction event may comprise: issuing a control instruction to a measurement device to cause the measurement device to acquire measurement data pertaining to the patient.

Additionally or alternatively, the triggering of a patient interaction event may comprise generating a prompt at a user interface to prompt a user to acquire measurement data using a measurement device.

In some embodiments, the system may further include functionality for receiving a patient interaction feedback signal indicative of successful completion of a patient interaction event, for example from a patient interaction device or from a user interface.

Another aspect of the invention is a method. The method may comprise accessing a workflow datastore storing a plurality of clinical workflow specifications, each workflow specification defining a plurality of workflow steps. The workflow steps may be linked by a process flow structure in the workflow specification. Each workflow step may be associated in the specification with a set of workflow step properties, wherein, for at least a subset of workflow steps, the workflow step properties include an indication of measurement data input requirements for the step and an indication of a patient measurement device to be used for acquiring the measurement data.

The method may further comprise accessing a measurement device inventory recording a list of available patient measurement devices for use in acquiring measurement data, wherein each measurement device is labelled with associated data acquisition capabilities and labelled as being a device which is either for on-site use or off-site use.

The method may further comprise generating one or more workflow step transformation suggestions, each step transformation suggestion indicating at least one possible step of a workflow specification which may be transformed from an on-site step to an off-site step. The generating of a workflow step transformation suggestion may comprise the following. For each of one or more workflow steps of one or more workflows which are currently associated with use of an onsite device: reading the input data requirements associated with the step; comparing the input data requirements against data acquisition capabilities of one or more measurement devices listed in the inventory; identifying an offsite measurement device in the inventory for which the data acquisition capabilities match the input data requirements; and generating a workflow step transformation suggestion indicative of the identified offsite measurement device from the inventory, and the workflow step and workflow for which the device has been identified.

The method may further comprise controlling a graphical user interface to provide an indication of generated workflow step transformation suggestion(s).

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 outlines steps of an example method in accordance with one or more embodiments of the invention;
Fig. 2 is a block diagram of an example processing device and system in accordance with one or more embodiments of the invention;
Fig. 3 schematically illustrates a processing flow according to one or more embodiments of the invention;
Fig. 4 shows a block diagram of an example system in accordance with one or more embodiments of the invention; and
Fig. 5 shows a block diagram of a further example system in accordance with one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for generating proposed modifications to clinical workflows for transferring monitoring steps which are, by default, performed on-site in a clinical setting such as a hospital to an off-site setting such as the home of the patient, a remote clinic, or a nursing home.. To this end, it is proposed to review workflow steps of a set of workflows and identify steps which involve acquisition of measurement data for a patient and to identify off-site, portable measurement devices from an inventory list whose data acquisition capabilities are sufficient to acquire the needed measurement data for that step. A corresponding workflow step transformation suggestion is generated.

In some embodiments, alarming settings associated with workflow steps which are transformed to use off-site measurement devices may be modified to account for the change to an offsite measurement device. For example, an offsite measurement device may be operable to acquire data with a lower degree of precision or with a wider confidence interval, or may acquire data at a lower sampling frequency, and/or transfer of measurement data from the device to the clinical decision support system which hosts the alarms may be only intermittent. In such circumstances, modification of the settings for the alarms may be advisable to avoid false alarms or to guard against potential missed alarms.

For example, in accordance with at least one set of embodiments, the system may include a processing device which receives as input:
a set of clinical workflows, e.g. hospital protocol workflows, and measurement devices normally used for each of those workflows, together with minimum data requirements recited in the workflows; and
a list or database of portable measurement devices, including their specifications, where the specifications for each device indicate at least the data the device is able to acquire and preferably quality characteristics of the data.

The system may use this input to match on-site (e.g. in-hospital) devices and offsite (e.g. non-hospital) devices capable of acquiring an equivalent measurement.

The system may generate as output, a measurement device or software suggestion.

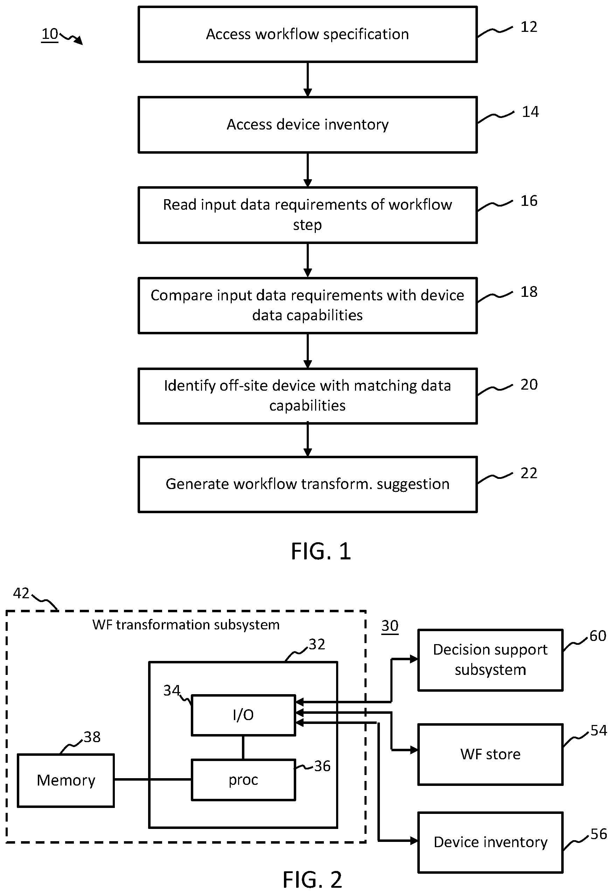

Fig. 1 outlines in block diagram form steps of an example method according to one or more embodiments. The steps will be recited in summary, before being explained further in the form of example embodiments.

The method 10 comprises accessing 12 a workflow datastore (or workflow store) storing a plurality of clinical workflow specifications. Each workflow specification defines a plurality of workflow steps. The workflow steps may be linked by a process flow structure, i.e. a logic and/or ordering. The logic progression structure may for example include a branching logic structure, for example where progression through steps of the workflow specification depend upon one or more criteria. For example, the one or more criteria may include receiving feedback indicative of successful completion of a current or previous workflow step, and/or based on values of one or more patient clinical parameters.

Each workflow step may be associated in the specification with a set of workflow step properties, wherein, for at least a subset of workflow steps, the workflow step properties include an indication of measurement data input requirements for the step and an indication of a patient measurement device to be used for acquiring the measurement data. For example, a workflow step may comprise acquiring input measurement data and comparing values of a measurement quantity represented by the measurement data against one or more criteria, e.g. thresholds. Alarm criteria may be defined, against which values of the received measurement data may be compared in order to determine whether an alarm condition has been reached.

The method 10 further comprises accessing 14 a measurement device inventory recording a list of available patient measurement devices for use in acquiring measurement data. Each measurement device may be labelled with associated data acquisition capabilities. Each device may be labelled as being a device which is either for on-site use or off-site use. For example, on-site use may mean on-site at a clinical site, for example a hospital. Offsite may mean away from the clinical site, e.g. at a home environment of a patient.

The method may further comprise generating one or more workflow step transformation suggestions, each step transformation suggestion indicating at least one possible step of a workflow specification which may be transformed from an on-site step to an off-site step. This means changing the measurement device assigned to the step from an on-site device to an offsite device.

This process may comprise, for each of one or more workflow steps of one or more workflows which are currently associated with use of an onsite device:
reading 16 the input data requirements associated with the step;
comparing 18 the input data requirements against data acquisition capabilities of one or more measurement devices listed in the inventory;
identifying 20 an offsite measurement device in the device inventory for which the data acquisition capabilities match the input data requirements; and
generating 22 a workflow step transformation suggestion indicative of the identified offsite measurement device from the inventory, and the workflow step and workflow for which the device has been identified.

The method may further comprise controlling a graphical user interface to provide an indication of generated workflow step transformation suggestion(s).

The method may further comprise implementing the transformation suggestion by modifying the measurement device assigned to the relevant workflow step of the relevant workflow and storing a record of the transformed workflow, either in the workflow store or elsewhere, e.g. in a working memory of a decision support system with which the workflow transformation subsystem is operatively coupled.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the processing device is shown as being comprised by a workflow transformation subsystem 42. For example, the workflow transformation subsystem may be configured for generating the one or more workflow step transformation suggestions using the process outlined above, or in accordance with any embodiment described in this disclosure.

In the illustrated example of Fig. 2, the system 30 further comprises a decision support subsystem 60, details of which will be discussed later.

In the illustrated example of Fig. 2, the system 30 further comprises a workflow store 54 ("WF store") which stores a plurality of clinical workflow specifications, each workflow specification defining a plurality of workflow steps linked by a process flow structure. Each workflow specification stored in the datastore may correspond to a particular clinical workflow. There may be more than one workflow specification associated with each possible workflow, e.g. where there are different versions of a same workflow, e.g. different ways to achieve the same workflow outcome. In this case, each workflow specification may be indexed in the workflow datastore 54 with a workflow ID which uniquely identifies the workflow to which the workflow specification corresponds.

Each workflow step may be associated in the workflow specification with a set of workflow step properties. For at least a subset of workflow steps, the workflow step properties include an indication of measurement data input requirements for the step and an indication of a patient measurement device to be used for acquiring the measurement data.

In the illustrated example of Fig. 2 the device further comprises a measurement device inventory 56 recording a list of available patient measurement devices for use in acquiring measurement data. The measurement device inventory may be a database or other data object. Each measurement device may be labelled with associated data acquisition capabilities. Each measurement device may be labelled as being a device which is either for on-site use or off-site use.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

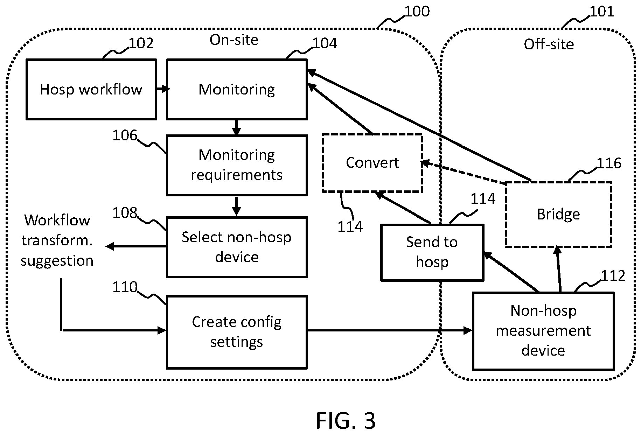

Fig. 3 outlines schematically the basic logic or processing flow implemented in accordance with at least a subset of embodiments of the invention. The processing flow illustrates the process for transforming a workflow step from on-site execution to off-site execution. The workflow step may be a step of acquiring measurement data pertaining to a patient. For example, measurement data may be measurement data of one or more biological parameters, for example one or more physiological parameters, for example one or more vital signs. Explicit examples of measurement data, and the associated measurement device for acquiring the data include the following.

Heart Rate: This can be acquired using a pulse oximeter or an electrocardiogram (ECG). The measurement device typically has sensors for placement on the patient's skin to detect electrical impulses or blood flow changes to calculate the heart rate.

Blood Pressure: A sphygmomanometer may be used comprising an inflatable cuff for wrapping around the arm of the patient.

Respiratory Rate: Spirometers or respiration rate monitors can be used. These devices measure the breaths per minute by detecting the rise and fall of a patient's chest or abdomen. A pulse oximeter may also be used.

Blood Oxygen Saturation: Pulse oximeters are devices that measure the oxygen saturation level in the blood. These may clip onto a patient's fingertip and use light reflection to determine the amount of oxygen in the blood.

Blood Glucose Levels: Blood glucose meters or glucometers are used. A small drop of blood, obtained by pricking the skin with a lancet, is placed on a disposable test strip that the meter reads and uses to calculate the blood glucose level.

Body Temperature: Thermometers can be digital or mercury-based and can be used orally, rectally, axillary, or tympanically. Infrared thermometers and temporal artery thermometers can also be used.

Electroencephalogram (EEG): This measures electrical activity in the brain. It involves placing electrodes on the scalp to detect brainwave patterns.

The process flow in Fig. 3 is schematically illustrated as being divided between an on-site location 100, e.g. in a hospital, and an off-site location 101, e.g. at a patient's home. An example hospital workflow 102 is schematically illustrated. At least one step of the workflow may call for monitoring 104 of at least one biological parameter of a patient using a particular measurement device. The default measurement device may be an on-site (e.g. hospital) measurement device. To enable the workflow step to be instead performed at an offsite location (e.g. the home of the patient), monitoring requirements 106 pertaining to the relevant workflow step are identified. Monitoring requirements include a type of measurement data to be acquired and optionally one or more further characteristics of the data such as data quality parameters, and/or sampling frequency. The measurement device inventory database is then consulted to identify an offsite (e.g. non-hospital) measurement device 108 which has data acquisition capabilities which match the data input requirements of the relevant workflow step. The offsite measurement device is for example a portable measurement device which can be used at a home environment. A corresponding workflow transformation suggestion may be generated and output, for example to a user interface for presentation to a user. A user may accept or select the workflow transformation suggestion, responsive to which, configuration settings 110 for the offsite measurement device may be determined and applied to the device. For example, these may indicate one or more of: a type of measurement to acquire (if more than one is possible), a frequency of measurement acquisition, a mode of data communication/transmission, a frequency of data transmission to a data collection server.

At the offsite location 101, the offsite measurement device 112 acquires measurement data. It may acquire measurements at a frequency defined by the configuration settings. The offsite measurement device may establish a communication channel with a patient monitoring module of a decision support system at the clinical site 100 for transferring acquired measurement data. The patient monitoring module may track measurement values of the biological parameter being measured. The patient monitoring module may include alarm functionality for triggering one or more alarms responsive to measurements meeting pre-defined alarm criteria.

The data transfer 114 from the offsite measurement device 112 to the on-site location may be performed quasi-continuously, i.e. the offsite measurement device 112 communicates a real-time data stream to the patient monitoring module at the clinical site. Alternatively, measurement data may be transferred batch-wise, wherein batches of measurement samples spanning defined time windows are locally stored in a local memory of the offsite device before being transferred to the patient monitoring module.

Data transfer to the hospital monitoring system can be performed directly by the device itself or via an indirect connection bridge 116 which can send the information to the hospital. For example, a bridge device may be a mobile computing device such as a smartphone, or may be a dedicated communication hub. Optionally a data filtering step may be included for reducing the measurement data to the most relevant subset of data, such that only the required monitoring data is transferred towards the hospital. This reduces network traffic.

Optionally, a transfer function may be applied to the offsite measurement data to perform conversion 114 of the measurement data, to improve a match between the measurement data obtained by the offsite measurement device and the data input requirements of the relevant workflow step 102. By way of example, conversion may comprise performing baseline correction. By way of further example, conversion may comprise performing unit transformation. By way of further example, conversion may comprise gain correction or amplitude correction. By way of further examples, conversion may comprise noise filtering, resampling, adding annotations/labelling, removing parts of the data, generating parts of missing data, and/or morphological adaptations.

Baseline correction in this context refers to a process of adjusting the measurement data to remove background signals, noise, and/or consistent offsets that are not relevant to the actual measurement. In this way, it has the effect of ensuring that the starting point, or baseline, of the transferred dataset is at zero or another defined level. Other examples of data conversion steps that might be performed include any one or more of: normalization, smoothing, filtering, calibration, linearization, unit conversion, data resampling, discretization, and de-trending.

The transfer function may be pre-determined. The transfer function may be identified using a lookup table in some embodiments. The transfer function may alternatively be determined in real time in some examples.

In some embodiments, alarm criteria used in performing patient monitoring at the hospital may be adapted to match the data acquisition capabilities of the offsite measurement device. For example, the clinical decision support subsystem 60 may include patient status monitoring functionality utilizing the received measurement data, as mentioned above (and to be discussed in greater detail to follow). For example, a duration alarm (which triggers if a certain measurement remains above a threshold for a defined time period) may need to be modified if the sampling frequency of the offsite measurement device is low, or if the offsite measurement device only transfers data in batches, so that there is a time Interval between each set of new data.

The hospital monitor system, monitors (via the smart alarms) if an alarm is activated and based on the alarm type can determine if a patient needs care. A response action can for example be to initiate a (video-) call with the patient to obtain more diagnostic information or to send (professional) care to the patient.

For example, in operation, a patient may be addressed to a specific workflow or protocol based on clinical signs and/or diagnosis. One of the steps of the workflow may include active monitoring or surveillance of one or more biological parameters or measurements. However, it may occur that, in particular circumstances, home monitoring is considered preferable for the patient. This might be due to high burden at the hospital, a relative low chance of alarms, a long time period required for monitoring, or improved patient status outside of the hospital.

As explained above, the data input requirements for the workflow step are recorded in the workflow datastore, and these may include requirements as to data type, data quality, or other data characteristics. These may be requirements needed for alarms to correctly run. From these, a suitable home-based monitoring device may be identified. If needed, a setup instruction transfer can be provided to setup configuration settings of the non-hospital device. The non-hospital monitoring measurement can be performed, data can be uploaded to the hospital, and depending on the monitoring requirements this transfer of data can be live or via intervals. This enables use of a non-hospital measurement device to be used in patient monitoring, while adhering to the hospital monitoring requirements.

One step of the implemented method is comparing input data requirements of a given workflow step with data acquisition capabilities of a given off-site measurement device.

Data input requirements may include requirement pertaining to any one or more of the following: data type, data quality, data sampling rate, device sensitivity, device measurement precision, device error correction functionality.

By way of one illustrative example, a simple requirement might include the capability to acquire measurements of a particular parameter continuously or quasi continuously (i.e. high sampling rate, as defined by a minimum sampling rate threshold). This may be needed for a given workflow step for example to enable a duration alarm to be implemented in which an alarm is triggered when a measured parameter remains outside of a given normal range for *x* amount of time. In such a case, it is essential that the off-site device be able to acquire measurements at a sampling rate which is significantly shorter than the time window x which is used to assess the alarm condition. If a given off-site measurement device cannot do this, it may be deemed unsuitable for the given workflow step.

With regards to the off-site measurement devices, these may be devices which are owned by the patient themselves, e.g. a smartwatch or other activity tracker, or for instance a device kept by the hospital which can be lent to the patient to use at home. In general, these may be portable devices.

By way of non-limiting example, off-site measurement devices might include:
ECG monitoring devices: Portable ECG devices are available that can be used at home to monitor a patient's heart rhythm and detect any irregularities. This can be particularly useful for patients with heart conditions who need frequent ECG monitoring.
Blood glucose monitoring devices: Patients with diabetes can use portable blood glucose monitors to check their blood sugar levels at home. This can save time and reduce the need for frequent visits to the hospital or clinic.
Blood pressure monitoring devices: Home blood pressure monitors are available that allow patients to measure their blood pressure at home. This can be useful for patients with hypertension who need frequent monitoring.
Respiratory monitoring devices: Patients with respiratory conditions such as asthma or COPD can use portable spirometers to monitor their lung function at home. This can help detect changes in lung function early and allow for early intervention.

As mentioned above, in some embodiments, alarm criteria used in performing patient monitoring at the hospital may be adapted to match the data acquisition capabilities of the offsite measurement device. For example, adaptations to alarm criteria might include: having earlier triggers, allowing for more false positives, and/or allowing sufficient travel time to hospital.

In some embodiments, the required modifications to the alarm criteria may be determined based on comparison between the input data requirements of the workflow step and the data acquisition capabilities of one or more measurement devices listed in the inventory. There may be a pre-determined database, lookup table or transfer function which permits transformation of initial alarm criteria to new alarm criteria based on results of a comparison between the input data requirements of the workflow step and the data acquisition capabilities of the relevant one or more measurement devices listed in the inventory.

According to one or more further examples, a set of one or more mathematical formulae may be pre-stored in a memory and each adapted to receive on-site alarm settings as input, and generate appropriate off-site alarm settings as output. Thus, in this example, the comparison between the input data requirements and the data acquisition capabilities of the off-site measurement device need not be used to reconfigure the alarm settings. For example, there may be one such algorithm for each different possible off-site measurement device in the inventory. By way of further example, there may be one such algorithm per combination of on-site measurement device and off-site measurement device.

By way of one illustrative example, this may be achieved by changing the alarm thresholds based on pre-set percentage. Of course, other types of calculations are also possible.

In the event that an offsite measurement triggers an alarm, this information might be first communicated to the hospital (e.g., tele-monitoring center), without directly alerting the patient. A response action may then be determined by a clinician at the hospital. This may involve contacting the patient to check the patient's status. It may involve updating the monitoring and measurement options (e.g., changes to alarm settings, changes to the measurement device).

With regards to the offsite measurement device, different options are possible. There exist dedicated portable measurement devices for measuring specific parameters. For example, the Philips MCOT-- Mobile Cardiac Outpatient Telemetry is operable to perform arrhythmia detection. However, a disadvantage with dedicated measurement devices is poor flexibility. More preferably, the offsite measurement device may be a generic measurement device, by which is meant a measurement device able to acquire a plurality of different measurement types. Examples of generic measurement devices which might be employed include for example: wearable devices such as smart watches or smart patches, or smart home devices (e.g. speech interfaces, video monitoring devices), or smart health devices such as portable blood analysis devices, urine analysis devices, saliva analysis devices.

Another example of a generic home-based device may be a personal computing device such as a smartphone. Here, a software application may be installed on the smartphone which is configured to control hardware features of the smartphone to acquire one or more measurements. Such measurements could include for example heart rate or blood oxygen measurements, e.g. using a PPG optical sensing arrangement included in the smartphone. Other options include camera based sensing or analysis, e.g. capturing and analyzing facial expressions. Other options include motion sensing, e.g. tracking activity of the user via an accelerometer of the device. Other options include acquiring input measurement data via the user interface of the smartphone based on prompting a user to answer questions or perform tasks. For example, the user may be asked to interact with one or more questionnaires as a means for data collection, or may be asked to play games, as a means for collecting the relevant user information, such as response time, cognitive capability, and/or the user may be asked to interact with specific user interfaces, or agents as a means to collect relevant healthcare data such as information about activities of daily living (nutrition, toilet visits, sleep), alertness, medication intake, etc.

An example use case is for arrhythmia detection.

Arrhythmia can be diagnosed in a hospital (on-site) setting using an electrocardiogram. Based on the findings, a treatment plan can be made. One of the treatments can be drugs or the correction of the heartbeat by a catheter procedure or surgery to implant a cardiac device. Regular check-ups in the hospital are often needed to determine if the treatment has effect.

Instead of use of an ECG in the hospital, home monitoring devices such as a heartbeat monitor and a SpO2 sensor can be used. This enables the diagnosis of arrhythmias and determination of a cause. The hospital monitoring requirements can be set-up as configuration settings for the home monitoring device. Although dedicated at-home arrhythmia monitoring solutions exist, these cannot be easily configured to match the hospital-specific protocol requirements. The use of more generic devices allows for more flexible configuration setting adjustments. To enable this, the system of the present invention provides means for automatically identifying measurement devices which may be suitable for use in implementing a given workflow step.

As discussed above, the decision support subsystem 60 may include alarm functionality for monitoring received patient monitoring data and comparing received measurements against alarm criteria to assess whether an alarm condition is breached. As mentioned above, the alarm conditions may be adjusted in accordance with some embodiments of the present invention to account for potential differences in the data acquisition properties or capabilities of an off-site measurement device.

Various types of alarms can be implemented in a clinical setting, as will be known by the skilled person. By way of non-limiting example, alarm types may include for example a peak alarm, a duration alarm, or combination of parameters alarm. With regards to a peak alarm, this type of alarm triggers when a monitored parameter (such as heart rate, blood pressure, or oxygen saturation) reaches a threshold value that is considered too high or too low. The 'peak' refers to the parameter exceeding or falling below a set value. For instance, if a patient's heart rate goes above 120 beats per minute or falls below 50 beats per minute, a peak alarm might sound to notify the healthcare team.

With regards to a duration alarm, this alarm is based on the duration or length of time a parameter remains outside a predefined range. Rather than triggering immediately when a threshold is breached (as with a peak alarm), a duration alarm might be set to sound only if the parameter remains outside the acceptable range for a set period, such as 10 minutes or more. This helps reduce false alarms that could be caused by brief and non-significant changes in a patient's status.

With regards to a combination of parameters alarm, this type of alarm considers multiple parameters simultaneously. It triggers when a specific combination of conditions pertaining to the multiple parameters is met. For instance, an alarm might sound if both a patient's heart rate is above a certain level and their oxygen saturation falls below a threshold. By considering multiple parameters, this type of alarm can provide a more comprehensive view of a patient's status, potentially identifying more complex or multifaceted issues that may not be identified if monitoring a single parameter.

To aid in understanding of the invention, there will now be presented a description of an example system architecture/environment for use in clinical decision support, within which the proposed workflow transformation method may advantageously operate.

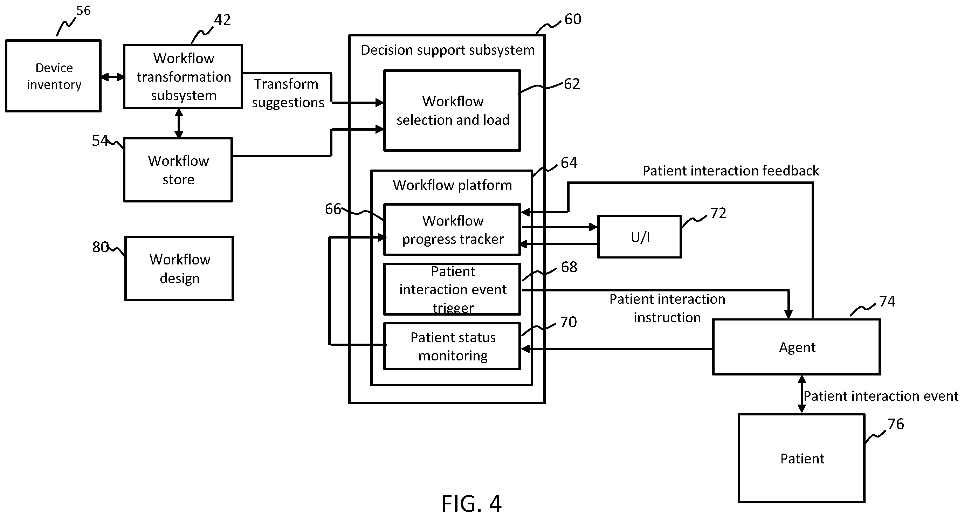

An example system is shown in Fig. 4. A system comprising any selected combination of one or more of the components of the system of Fig. 4 may be provided as an aspect of the invention. The illustrated system includes a decision support subsystem 60. The decision support subsystem 60 is for providing clinical decision support to a user, for example real-time clinical decision support.

At least one functionality provided by the clinical decision support subsystem may be the hosting of clinical workflows. The system of Fig. 4 includes a workflow hosting platform 64. Hosting of a clinical workflow may comprise tracking progress through steps of the workflow specification associated with the workflow and/or managing execution of steps of the workflow specification by generating patient interaction instructions for causing or prompting execution of a patient interaction event by an agent, where the agent may be a human agent or a machine (hardware/software) agent.

The workflow hosting platform 64 includes a workflow progress tracker 66 for tracking progress through steps of each live workflow. The workflow progress tracker may be operatively coupled with a user interface 72 and configured to output a workflow progress status indictor to a user interface indicative of progress through the steps of the clinical workflow. One or more of the steps of a clinical workflow being hosted may indicate a need for a patient interaction, for example acquiring information about a patient state, or modifying a patient state, e.g. changing or configuring an element of treatment. Acquiring information about a patient state may mean acquiring one or more biological/physiological measurements pertaining to the patient, where this might be done using one or more patient measurement devices.

The workflow hosting platform 64 may further comprise a patient interaction event trigger module 68 for generating a patient interaction instruction for causing or prompting execution of a patient interaction event by an agent 74, where the agent may be a human agent or a machine (hardware/software) agent. Fig.4 shows the communication of the patient interaction instruction schematically. If the agent is a human, the instruction generated by the patient interaction event trigger may take the form of a user interface prompt communicated to the user interface 72 for presentation to a human agent, instructing or guiding the user to perform a specified patient interaction event. If the agent is a machine, e.g. a patient sensing/measurement device or patient therapeutic device, the instruction may comprise a machine (control) instruction for causing the device to perform a patient interaction event such as acquiring measurement data or administering or adjusting a therapy to the patient.

The workflow progress tracker 66 may be arranged to receive patient interaction feedback generated by the agent 74 following execution of a patient interaction event, for example confirmation that a patient interaction event has been successfully completed. This can be used in tracking progression through the steps and/or logic structure of the workflow specification of a workflow being hosted. If the agent is a human agent, the patient interaction feedback may be received at the workflow progress tracker from the user interface 72 as user input information provided to the user interface by the human agent. If the agent is a machine agent, the patient interaction feedback information may be received by the workflow progress tracker 66 from the machine agent itself 74 which may generate a feedback signal responsive to successful completion of the patient interaction event.

In the example of Fig. 4, the workflow hosting platform 64 further comprises a patient status monitoring module 70 for monitoring a patient status. Thus may comprise monitoring one or more sensor measurements acquired for the patient. It may comprise receiving update information via a user interface 72 by a human agent relating to patient status. The receipt of the patient status information at the patient status monitoring module 70 is shown schematically in Fig. 4 as being received by the agent 74. If the agent is a human agent, the information may be received from the user interface 72 as user input information by the human agent. If the agent is a machine agent, the information may be received directly from the machine agent. Examples of human agent originating information might be a visual observation made by a human clinician or another physical examination result.

The patient status monitoring module 70 may communicate with the workflow progress tracker 66 and the workflow progress tracker may update a tracked progress through the steps of a hosted workflow based on information received from the patient status monitoring module. For example, reaching a threshold level of a certain physiological parameter may be a trigger condition for progressing to a next step or a different branch of the workflow specification in some examples.

The patient status monitoring module 70 may also monitor one or patient alarms associated with measurement data being received from patient measurement devices. This may comprise continuously monitoring one or more alarm criteria pertaining to one or more of the measurement devices to detect if and when an alarm condition exists.

Thus, in general terms, the workflow hosting platform 64 may perform live or real-time hosting of one or a plurality of workflows. Hosting of a workflow may comprise tracking progress through a set of steps of the workflow linked by a logical progression structure. For a current step of a workflow being hosted, the workflow platform may generate an output, e.g. an instruction, for triggering or prompting execution of one or more patient interaction events comprising obtaining information about a current patient status or administering or adjusting a therapeutic action associated with a patient. Feedback may be received indicative of successful completion of a patient interaction event following issuance of the patient interaction event instruction. The progress through the workflow may be updated based thereon. Patient status may also be monitored, e.g. values of one or more physiological parameters, or clinician observations, and this information may also be used to update a progress status relative to the workflow specification. Feedback regarding progress status relative to the workflow specification may be output regularly, e.g. continuously, to a user interface 72.

With regards to the workflows, the decision support system 60 may further include a workflow selection and loading module 62. The workflow selection and loading module may be communicatively coupled with the workflow store 54 which stores a plurality of different workflows, each associated with a respective workflow specification. One or more workflows may be selected and loaded from the workflow store and passed to the workflow hosting platform for hosting.

Fig. 4 illustrates the workflow transformation subsystem 42 previously discussed. The workflow transformation subsystem 42 may receive a workflow transformation request message from the decision support subsystem 60, e.g. the workflow selection and loading module 62. The workflow transformation subsystem may output a transformed workflow to the decision support subsystem 60 following execution of a workflow transformation method in accordance with any of the examples or embodiments described in this document. Alternatively, the workflow transformation subsystem 42 may run automatically, e.g. in the background of the main system functions of the decision support subsystem 60, and deliver workflow step transformation suggestions to the workflow selection and loading module 62.

Fig. 4 also schematically illustrates an optional element of the system implementation comprising design 80 and input of the workflows which populate the workflow store 54. For example, a human user may design and generate each of one or more workflow specifications. Each workflow specification comprises may comprise a sequence of steps linked by a logic progression structure. The logic progression structure may for example include a branching logic structure, for example where progression through steps of the workflow specification depend upon one or more criteria. For example, the one or more criteria may include receiving feedback indicative of successful completion of a current or previous workflow step, and/or based on values of one or more patient clinical parameters. Embodiments of the invention may thus include a process of a user inputting one or more workflow specifications to the workflow store. Embodiments may include a process of a user designing one or more workflow specifications. The user may design the workflow specifications for example in accordance with a known clinical protocol and/or a known clinical practice guidelines.

Above has been presented a description of the general architecture and logic of an example decision support environment within which embodiments of the present invention may operate. As will be recognized, embodiments of the present invention primarily relate to selection and/or configuration of patient measurement devices for use off-site, e.g. at home. Thus, in this context, the agents 74 referred to above for performing patient interaction events may include one or more off-site patient measurement devices for performing patient interaction events in the form of acquiring biological measurements such as physiological parameter measurements pertaining to the patient. It will be appreciated that the same decision support subsystem 60 may be arranged to receive measurement data for a multiplicity of different patients receiving care, from a multiplicity of different data sources, which will typically include on-site measurement devices as well as off-site measurement devices.

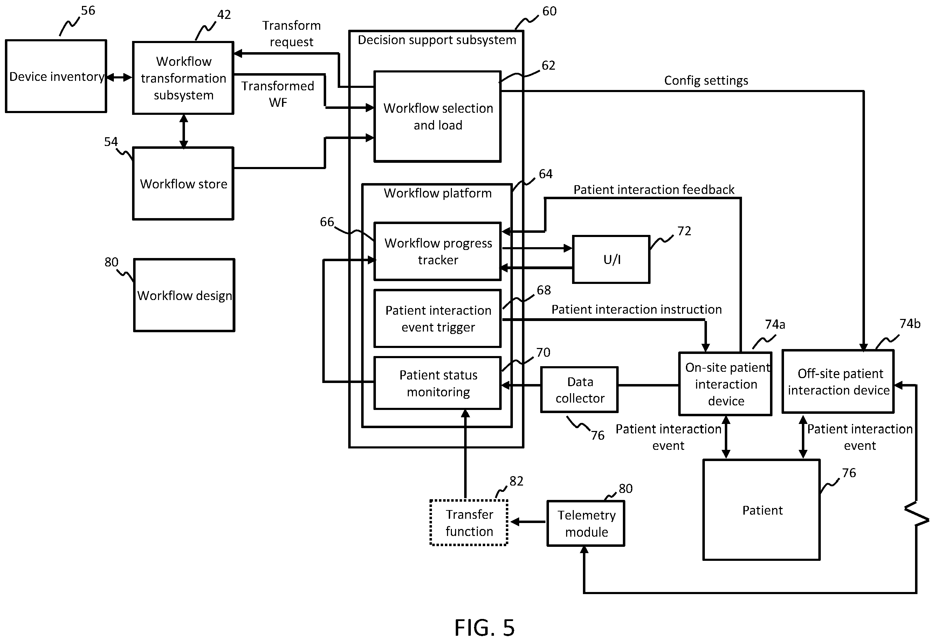

To illustrate the interaction of the decision support subsystem with off-site and on-site measurement devices, Fig. 5 schematically illustrates an example decision support architecture suitable for use with embodiments of the present invention illustrating on-site and off-site measurement devices. This system may comprise the same features and functionality as described in relation to the system of Fig. 4, with the following described additions or modifications. Any combination of the components of the overall system of Fig. 5 may be provided as an aspect or embodiment of the invention.

In the example of Fig. 5, the agent 74 role is represented with two particular examples of possible agents, one an-site patient interaction device 74a, and one an off-site patient interaction device 74b. In the context of embodiments of the present invention, the off-site patient interaction device may be an off-site patient measurement device.

Also shown is an optional on-site data collector 76 for collecting data generated by the on-site patient interaction device, such as measurement data. For example, this may be a hub device which collects data from a plurality of different patient interaction devices and transfers it to the patient status monitoring module 70. For example, the data collector may convert received data to a uniform format suitable for receipt by the patient status monitoring device.

Also shown is an optional off-site data collector or telemetry module 80 for collecting data generated by the off-site patient interaction device, such as measurement data from off-site measurement devices. For example, this may be a hub device or server. The data collector or telemetry module 80 may be operable to communicate with offsite measurement devices 74b via a communication channel to collect measurement data acquired by one or more off-site measurement devices 74b. The telemetry module may be further configured to transfer measurement data acquired by off-site measurement devices 74b from the telemetry module to the decision support subsystem 60. For example, the data may be transferred to the patient status monitoring module 70. The telemetry module 80 may be arranged to receive data from a multiplicity of different off-site measurement devices, e.g. via an internet or other network link. Optionally, the received data may be passed through a transfer function 82 before being passed to the decision support subsystem 60, for one or more data conversion steps. Details relating to this optional feature have already been discussed above.

Although two data collecting modules 76, 80 are shown in the example system of Fig. 5, one 78 for collecting on-site data and one 80 for collecting off-site data, the function of these two modules could optionally be combined into a single data collection module in some embodiments.

Also illustrated in the example of Fig. 5 is the transfer of configuration settings to the off-site patient interaction device 74b. These may be generated by the workflow selection and loading module 62. Details relating to the generation of suitable configuration settings have already been discussed above.

Optionally, in addition to generating suitable configuration settings for the off-site patient measurement device 74b, also modifications to one or more alarm conditions associated with the measurements to be acquired by the off-site measurement device may be made. These may for example be determined by the workflow transformation subsystem 42, and transferred to the patient status monitoring module 70 (e.g. by the workflow selection and loading module 62) for implementation for data received by the off-site measurement device. The patient status monitoring module may recognize data that is received from the off-site device e.g. based on device ID metadata encoded in measurement data received remotely from the off-site measurement device.

In operation, the off-site measurement device 74b may be setup while located on-site by generation and transfer of suitable configuration settings. The patient may then be given the measurement device 74b to take away to an off-site (e.g. home) environment. At the off-site environment, measurement data my be generated by the off-site measurement device 74b at a certain measurement acquisition rate, which may be continuous or quasi-continuous, or intermittent. Measure data generated by the off-site device then must be transferred to the on-site decision support system 60. This can be performed by the off-site measurement device establishing a communication channel with the off-site data collector module or telemetry module 80. The data collection or telemetry module 80 receives the off-site measurement data and transfers it to the decision support subsystem 60, e.g. to the patient status monitoring module 70. As mentioned above, the patient status monitoring module 70 may also monitor one or patient alarms associated with measurement data being received from patient measurement devices. This may comprise continuously monitoring one or more alarm criteria pertaining to one or more of the measurement devices to detect if and when an alarm condition exists.

Various further optional features which may be implemented in any of the embodiments of this invention will now be discussed.

In some embodiments, upon implementing a suggested workflow step transformation to a given workflow, one or more further steps of the same workflow may be modified. This may be for example to account for the change from an on-site measurement to an off-site measurement. For example, in some embodiments, the workflow transformation module may have functionality for checking an impact on the remainder of a workflow of a given workflow step transformation suggestion for that workflow.

For example, as discussed above, each workflow step may be associated with a set of data input requirements and/or may be associated with certain patient interaction events. Functionality may be implemented to check or verify if the corresponding input data requirements, or the input data collection requirements, have been satisfied. If the data input requirements have not been met, then additional data might be collected (e.g. a new type of data, or additional data of the same type - for example data collected over a longer time period, of a higher quality, of a higher sampling rate, and/or of a higher signal to noise ratio).

For example, in some cases, the workflow transformation module may add one or more additional workflow steps to the workflow, modify one or more existing workflow steps, remove one or more workflow steps and/or change an order of the workflow steps. For example, one or more workflow steps might need to be adapted due to the change to a non-hospital measurement step. For example, steps in the workflow which are to remain as on-site measurement steps might be modified so that on-site measurements are obtained with a measurement device and/or measurement device settings the same as those which will be used for the off-site measurements to ensure comparability of data. In other words, hospital measurement devices may be adapted to match non-hospital measurement devices.

In some embodiments, the workflow step transformation suggestion may be a context-dependent transformation suggestion. For example, the transformation suggestion may encode a conditional logic, wherein the transformation suggestion is conditional on one or more variables pertaining to a context of the patient or of real-time demand on the clinical team. For example, the workflow transformation suggestion may encode a plurality of different transformation options which have a priority order or are conditional on circumstance.

For instance, an example may be considered of a workflow which comprises four workflow steps. The workflow transformation subsystem 42 may read the workflow steps from the workflow store 54. Based on analysis of the data input requirements as discussed above, the transformation subsystem 42 recognizes that: task 1 can only be performed in a hospital setting; task 2 is more preferably performed in a hospital setting due to data quality requirements but can be performed in an off-site setting, at the risk of lower quality data; step 3 can be performed in a home environment without any loss of data reliability, step 4 also can be readily performed in a home environment. The workflow step transformation suggestion comprises a suggestion that steps 3 and 4 be performed using off-site measurement devices. However, additionally, the workflow transformation suggestion also comprises a suggestion that the measurement device used in step 1 be changed to a secondary device whose output data matches the output data of the off-site measurement device. This thereby improves comparability of data. Furthermore, for step 2, a priority ordering, or context conditionality, is encoded in the suggestion, wherein step 2 is by default performed in on-site but, in a high demand scenario in the hospital, this step could be transformed to an off-site step using an off-site measurement device.

As mentioned above, one optional functionality is the conversion or transformation of data received from an off-site measurement device to improve a match between the data received from the off-site device and data input requirements of a given workflow step. Further implementation options related to this functionality will now be discussed.

Ideally there is a direct match between the output data type and data quality of the on-site (e.g. hospital) device and off-site (e.g. non-hospital) measurement devices. However, in some cases, there may not be a direct match, in which circumstance one or more data transformation or conversion steps may be applied to match the output and quality of the data.

By way of example, in some embodiments, responsive to detecting a match in the measurement data type and a mismatch in the measurement quality characteristics between an on-site device and a proposed off-site device, the workflow transformation subsystem may be configured to determine a transfer function for transforming measurement data acquired by the off-site measurement device into transformed measurement data having data quality characteristics better matching the required characteristics.

An applied transformation function may be generic or personalised. A generic transformation function might include for example baseline or noise correction. In some cases, additional measurement devices may need to be added to enhance the data quality. Computing a suitable transfer function can be performed by acquiring a dual measurement, i.e. acquiring a plurality of measurements of a same patient at the same time using both the on-site and the off-site measurement devices and determining a transfer function which maps the off-site to the on-site measurement. This could be performed using a regression fitting for example.

A personalized transformation may instead be used which takes account of patient-specific factors, e.g. comorbidities of the patient which can influence the measurement, or medication which could affect the measurement output or baseline. A dual measurement could also be used in this case to determine the personalized transformation function.

Some particular examples are presented below.

For a given step in a workflow, the output data type; file format, baseline and data measurement range may be determined for the on-site and off-site devices.

By way of one example, by measuring an ECG trace for a same patient at a same time using both devices, a transfer function can be calculated which maps between the two measurement devices. This transfer function can made personalized when measured on an individual person or can be generalized when a group of patients is used.

For example, an ECG signal trace of the off-site device might have a lower signal strength (e.g. in absolute voltage terms) than the on-site device, although the morphology of the signal trace is the same. Thus, a transfer function might be applied which scales the amplitude of the off-site measurements (e.g. gain adjustment) to match the signal level of the on-site measurement device.

In some embodiments, patient-specific data may be used to customise the transfer function. At least a subset of this information may be received from a user mobile computing device (e.g. smartphone). This could include information about the user's clinical history, measurement quality that can be achieved by the user, and/or user measurement skills. Such information further be used to determine how the transformation can be done.

As mentioned above, in some cases, switching to an off-site measurement device may be coupled with modification of one or more other steps of the workflow for which the off-site measurement device is to be used. In some embodiments, such modifications to the other steps may optionally take account of patient-specific context. For example, in many cases, patients may have co-morbidities (e.g. other underlying diseases, low/high BMI or other abnormal factors) which may influence the measurement or the procedure itself. These patient-specific factors may be taken into account in the workflow transformation to for example change an order of protocol steps or additional measurements. Such patient-dependent modifications may be implemented using pre-determined rules, for example wherein different sets of modifications are made responsive to particular sets of one or more co-morbidities or other personal factors.

In some embodiments, an additional procedure may be implemented to check a competency of a patient or off-site caregiver (e.g. family/friends or tele-health assistant) in acquiring requirement measurement data at an off-site location. In the case that a step which has been recommended to be switched to an off-site setting cannot be competently performed by the patient or other off-site caregiver, the step transformation recommendation might not be implemented (so that the step is retained on-site) or may be modified so that the measurement step is performed in a clinical setting outside the hospital rather than a home setting.

For example, in some embodiments, the system may include functionality for performing a measurement acquisition check function. This may comprise for example receiving measurement data from an offsite measurement device indicated by a workflow transformation suggestion, during a trial measurement session; evaluating one or more pre-defined data quality characteristics of the data; and generating feedback based on the evaluation. The measurement acquisition check function may thereby provide an assessment of a competency of the person acquiring the measurement using the off-site device.

By way of example, the competency assessment could be performed manually or automatically. A manual check may comprise the patient or off-site caregiver performing the relevant measurement using the off-site measurement device under the supervision of hospital staff. In this case, the observing staff member determines whether the measurement was performed correctly. An automatic check may comprise the patient or off-site caregiver performing the measurement using the off-site measurement device unsupervised, and wherein the acquired measurement is compared with a corresponding hospital measurement device output to compare data quality characteristics. Analysis can be applied to determine whether the procedure was performed the right manner.

In some embodiments, functionality may be implemented to check input data requirements of a given workflow step are being met by data received remotely from an off-site measurement device in real-time during monitoring of a patient off site. The analysis for example may comprise assessing data quality, and data type. If data input requirements of measurement data being received do not match expected data input requirements, then the decision support subsystem may transmit an electronic message to the patient at the off-site location indicating that the measurement needs to be repeated. Furthermore, the decision support subsystem may be configured to detect measurements which are above or below a certain threshold. In this case, the system can automatically alert the patient, hospital or in critical cases even the ambulance.

In some embodiments, the decision support subsystem includes a graphical user interface which may be controlled to simultaneously provide, for a given workflow transformation suggestion, a visual representation of: the original workflow to which the suggestion relates, and the suggested workflow transformation. In other words, the system may show a dual representation in which both the original workflow and the transformed workflow are visualized. Using the dual representation, the physician is able to easily compare the protocols and observe constraints such as adapted steps in the protocol or changes in the measurement devices.

Additionally or alternatively, the graphical user interface may be controlled to present a representation of off-site measurement devices recorded in the inventory which are currently available.

In some embodiments, when a given workflow transformation suggestion is accepted by a clinician user, information indicative of the transformed workflow may be communicated to the patient or an off-site caregiver. For example, it may be communicated to a mobile computing device of the patient or off-site caregiver. The patient or off-site caregiver thus is provided notice of the additional measurement step which is to be performed by them at the off-site setting.

Embodiments of the invention may be advantageously applied to a wide variety of different use cases.

There are several health conditions where hospital-based measurements can be complemented with off-site, e.g. home-based, measurements, and thus for which embodiments of the present invention may provide significant advantage. By way of non-limiting example, these include the following
High blood pressure: People with hypertension may need to monitor their blood pressure regularly. In addition to measurements taken in a clinical setting, home-based measurements can provide additional data on blood pressure trends over time.
Diabetes: People with diabetes may need to monitor their blood sugar levels regularly. Home-based measurements of blood sugar can provide additional information to help manage the condition.
Sleep apnea: People with sleep apnea may be asked to do a home sleep study to monitor their breathing patterns and oxygen levels while they sleep. This can provide additional information to help diagnose the condition and determine the best course of treatment.
Heart disease: People with heart disease may need to monitor their heart rate and rhythm regularly. Home-based monitoring can provide additional data on heart function and can help detect irregularities that may require medical attention.
Pregnancy: Pregnant women may be asked to monitor their blood pressure, blood sugar levels, and fetal movement at home. This can provide additional data to help manage the pregnancy and ensure the health of both mother and baby.

By way of one non-limiting example, one use case may be where a patient might be discharged earlier because of the availability of home-based measurement and monitoring options is after a surgical procedure. After surgery, patients are typically monitored in the hospital for a period of time to ensure that they are recovering well and there are no complications. However, with the availability of home-based monitoring options, such as remote vital sign monitoring and wound care management, some patients may be able to go home sooner and continue recovery at home.

For example, after a minor surgery, a patient could be discharged from the hospital with a portable monitoring device that allows them to measure their vital signs, such as heart rate, blood pressure, and oxygen saturation, at home. The patient would also be provided with instructions on how to care for their wound and would be able to send updates on their recovery progress to their healthcare provider through a telemetry module such as a telehealth platform.

By using home-based monitoring options, the patient can continue to recover at home under the guidance of their healthcare provider, while freeing up hospital beds for patients who require more intensive care. This can also reduce the risk of hospital-acquired infections, which can be a concern for patients who are in the hospital for an extended period of time.

Another use case is follow-up for patients with chronic diseases (such as asthma, cancer, diabetes) which is often lifelong and managed by regular pre-scheduled hospital visits. These hospital visits may occur when the patient is well, and neither the patient nor the clinician finds these visits necessary. A home measurement system which is based on the clinical hospital process may avoid these unnecessary hospital visits.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (30) comprising:
a workflow datastore (54) storing a plurality of clinical workflow specifications, each workflow specification defining a plurality of workflow steps, wherein each workflow step is associated in the specification with a set of workflow step properties, wherein, for at least a subset of workflow steps, the workflow step properties include an indication of measurement data input requirements for the step and an indication of a patient measurement device to be used for acquiring the measurement data;
a measurement device inventory (56) recording a list of available patient measurement devices for use in acquiring measurement data, wherein each measurement device is labelled with associated data acquisition capabilities and labelled as being a device which is either for on-site use or off-site use;
a workflow transformation subsystem (42) for generating workflow step transformation suggestions, each step transformation suggestion indicating at least one possible step of a workflow specification which may be transformed from an on-site step to an off-site step, based on:
accessing (12) the workflow datastore;
accessing (14) the measurement device inventory;
for each of one or more workflow steps of one or more workflows which are currently associated with use of an onsite device:
reading (16) the input data requirements associated with the step;
comparing (18) the input data requirements against data acquisition capabilities of one or more measurement devices listed in the inventory;
identifying (20) an offsite measurement device in the inventory for which the data acquisition capabilities match the input data requirements;
generating (22) a workflow step transformation suggestion indicative of the identified offsite measurement device from the inventory, and the workflow step and workflow for which the device has been identified; and
controlling a graphical user interface to provide an indication of generated workflow step transformation suggestion(s).

2. The system of claim 1, where the workflow transformation subsystem (42) is further configured to, for at least one workflow step transformation suggestion:
replace the measurement device property of the relevant workflow step with the offsite measurement device identified from the inventory; and
output the modified workflow step.

3. The system of claim 1 or 2, wherein the system further comprises alarm monitoring functionality for monitoring received measurement data from one or more of the measurement devices and comparing it against one or more alarm conditions.

4. The system of claim 3,
wherein one or more of the workflows in the workflow datastore include at least one step of monitoring a first physiological parameter of a patient and include an indication of at least one alarm condition in relation to the physiological parameter; and
wherein the workflow step transformation suggestion includes a modification of the at least one alarm condition.

5. The system of claim 4,
wherein the modification of the at least one alarm condition is determined based on the comparison between the input data requirements of the workflow step and the data acquisition capabilities of one or more measurement devices listed in the inventory.

6. The system of any preceding claim,
wherein the system includes a data collection module (80), operable to communicate with offsite measurement devices (74b) via a communication channel to collect measurement data acquired by one or more off-site measurement devices; and
wherein the data collection module is configured to transfer measurement data acquired by off-site measurement devices from the data collection module to a decision support subsystem (60).

7. The system of any preceding claim,
wherein the system includes a plurality of off-site measurement devices (74b);
wherein each offsite measurement device includes functionality for exporting acquired measurement data via a direct or indirect communication channel with a data collection module of the system.

8. The system of any preceding claim, further comprising a data transformation module (82) for:
receiving measurement data acquired by an offsite measurement device (74b); and
applying a data transformation function for modifying the received data to improve a match between the received measurement data and data input requirements of at least one workflow step of a workflow.

9. The system of any preceding claim,
wherein the input data requirements for each workflow step include an indication of: a type of measurement data, and one or more data quality characteristics;
wherein the data acquisition capabilities recorded in the inventory for each measurement device include an indication of measurement data type and data quality characteristics;
wherein the comparing comprises comparing the required data quality characteristics against the data quality characteristics of each measurement device, and wherein, responsive to detecting a match in the measurement data type and a mismatch in the measurement quality characteristics, the workflow transformation subsystem is configured to determine a transfer function for transforming measurement data acquired by the off-site measurement device into transformed measurement data having data quality characteristics better matching the required characteristics.

10. The system of any preceding claim, further comprising a measurement acquisition check function, configured to:
receive measurement data from an offsite measurement device indicated by a workflow transformation suggestion, during a trial measurement session;
evaluate one or more pre-defined data quality characteristics of the data; and
generate feedback based on the evaluation.

11. The system of any preceding claim, further comprising a graphical user interface (72) controlled to simultaneously provide, for a given workflow transformation suggestion, a visual representation of: the original workflow to which the suggestion relates, and the suggested workflow transformation.

12. The system of any preceding claim, further comprising a workflow hosting platform (64) for hosting one or more workflows, the workflow hosting platform comprising functionality for tracking (66) progress through steps of each workflow being hosted.

13. The system of any preceding claim, wherein the system further comprises functionality for triggering (68) one or more patient interaction events specified by steps of a workflow for acquiring measurement data pertaining to a patient.

14. The system of claim 13, wherein the triggering a patient interaction event comprises:
issuing a control instruction to a measurement device to cause the measurement device to acquire measurement data pertaining to the patient; and/or
generating a prompt at a user interface to prompt a user to acquire measurement data using a measurement device.

15. A method, comprising:
accessing (12) a workflow datastore storing a plurality of clinical workflow specifications, each workflow specification defining a plurality of workflow steps, wherein each workflow step is associated in the specification with a set of workflow step properties, wherein, for at least a subset of workflow steps, the workflow step properties include an indication of measurement data input requirements for the step and an indication of a patient measurement device to be used for acquiring the measurement data;
accessing (14) a measurement device inventory recording a list of available patient measurement devices for use in acquiring measurement data, wherein each measurement device is labelled with associated data acquisition capabilities and labelled as being a device which is either for on-site use or off-site use;
generating one or more workflow step transformation suggestions, each step transformation suggestion indicating at least one possible step of a workflow specification which may be transformed from an on-site step to an off-site step, based on:
for each of one or more workflow steps of one or more workflows which are currently associated with use of an onsite device:
reading (16) the input data requirements associated with the step;
comparing (18) the input data requirements against data acquisition capabilities of one or more measurement devices listed in the inventory;
identifying (20) an offsite measurement device in the inventory for which the data acquisition capabilities match the input data requirements;
generating (22) a workflow step transformation suggestion indicative of the identified offsite measurement device from the inventory, and the workflow step and workflow for which the device has been identified; and
controlling a graphical user interface to provide an indication of generated workflow step transformation suggestion(s).
